# EUROPÄISCHE PATENTANMELDUNG

(11) **EP 1 544 615 A1**
(43) Veröffentlichungstag der Anmeldung: **22.06.2005**
(21) Anmeldenummer: 04106535.0
(22) Anmeldetag: 14.12.2004
(51) Int. Cl.: G01N 33/28

(54) **Verfahren und Vorrichtung zur Fehlergasüberwachung in flüssigkeitsgefüllten Hochspannungsanlagen**

(30) Priorität: 16.12.2003 DE 10359143; 10.11.2004 DE 102004054195
(71) Anmelder: Bräsel, Eckhard, 17493 Greifswald (DE)
(72) Erfinder: Bräsel, Eckhard, 17493 Greifswald (DE)
(74) Vertreter: Garrels, Sabine

(57) **Zusammenfassung**

Offenbart wird ein Verfahren und eine Vorrichtung zur Fehlergasüberwachung in luftatmenden flüssigkeitsgefüllten Hochspannungsanlagen, insbesondere eine als Fehlergasfrühwarngerät einsetzbare Messeinrichtung, mit der eine im Aktivteil des Transformators einsetzende Fehlergasemission lückenlos zuverlässig festgestellt werden kann. Das erfindungsgemäße Verfahren ist gekennzeichnet durch die Messung und Auswertung des Fehlergases Wasserstoff wahlweise
a) in dem aus dem Gasraum (4) des Ausdehnungsgefäßes (3) abgeleiteten Messgas oder
b) in dem bei einem Gasalarm des Gassammelrelais (2) aus diesem abgeleiteten Buchholzgas
in einer einheitlichen Online-Messeinrichtung (6) mit einem Wasserstoffsensor (22), wobei bei einem Gasalarm das Gassammelrelais (2) die Überwachung des Gasraumes (4) des Ausdehnungsgefäßes (3) unterbrochen, auf eine Messung und Auswertung auf das Fehlergas Wasserstoff in dem abgeleiteten Buchholzgas umgeschaltet und nach Vorliegen eines Prüfergebnisses auf die Überwachung des Gasraumes (4) zurückgeschaltet wird und bei einem Prüfergebnis "Fehlergas Wasserstoff" im Buchholzgas eine Vollanalyse durchgeführt wird.

## Beschreibung

### Technisches Gebiet

Die Erfindung betrifft ein Verfahren zur Überwachung von Fehlergasen in flüssigkeitsgefüllten Hochspannungsanlagen, deren Flüssigkeit über ein Ausdehnungsgefäß, vor dem ein Gassammelrelais angebracht sein kann, mit der atmosphärischen Luft in Kontakt steht, und eine entsprechende Vorrichtung.

### Stand der Technik

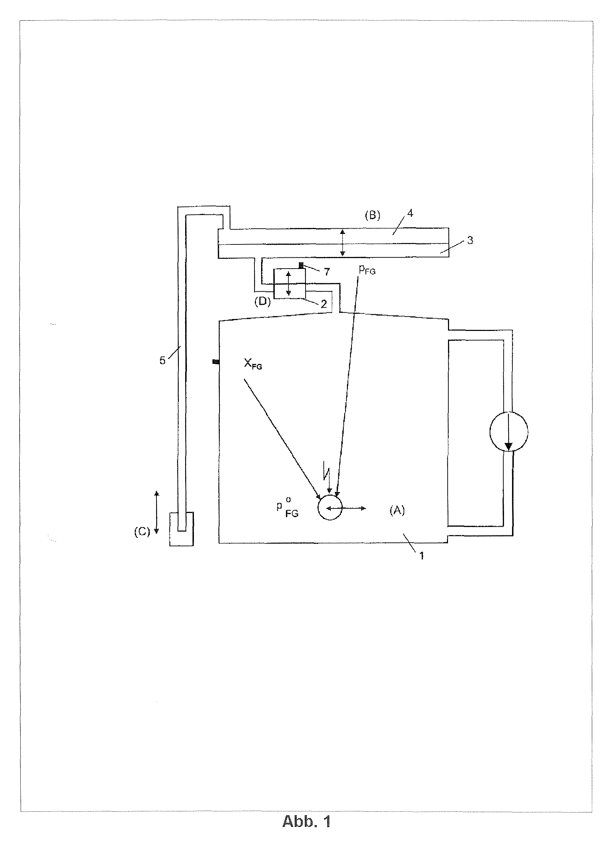

Das Grundprinzip der gasanalytischen Überwachung an einer flüssigkeitsgefüllten Hochspannungsanlage ist in Fig. 1 dargestellt. Lokal im Innern sich herausbildende elektrische bzw. thermische Anomalien erzeugen ab einer bestimmten Energiedichte Originalfehlergas in Blasenform, das mit der lufthaltigen Flüssigkeit in einen Gasaustausch (A) tritt, in deren Folge sich die Gasblasen entweder vollständig auflösen (x_{FG}) oder das Gassammelrelais (Buchholz-Relais) erreichen (p_{FG}) und einen Gasalarm auslösen. Das Originalfehlergas (p^{o}_{FG}) liegt weder gelöst noch als Buchholzgas vor, da Veränderungen eingetreten sind. Geringlösliche Fehlergase gehen aus der Flüssigkeit dadurch verloren, dass sie durch die Konvektion (B) mit der Flüssigkeit im Ausdehner über den Gasraum und die Atmungsleitung in die Atmosphäre gelangen. Im Buchholzgas dagegen gehen die gutlöslichen Fehlergase durch den Flüssigkeitskontakt (D) verloren. Nur für das Originalfehlergas gilt für Menge und Zusammensetzung ein gesicherter Zusammenhang zur Fehlerursache. Da das Originalfehlergas analytisch nicht zugänglich ist, müssen die analytischen Informationen von den Probenahmestellen (x_{FG} und p_{FG} ) auf das Originalfehlergas (p^{o}_{FG}) korrigiert werden. In DE 198 33 601 C 1 sind Verfahren und Vorrichtung beschrieben, die das gestatten. Dazu wird kontinuierlich nach dem Henry-Dalton'schen Gesetz ein Gleichgewichtsgas erzeugt und in diesem periodisch die Zusammensetzung bestimmt. Treten ungelöste Fehlergase im Buchholz-Relais auf, werden diese ähnlich dem Gleichgewichtsgas gehandhabt. So können mit repräsentativen Analysenwerten und den Zusammenhängen zum Gashaushalt zuverlässige Diagnosen erstellt werden.

Wegen der hohen Kosten einer Überwachung der Originalfehlergasemissionen zum Zwecke zuverlässiger Diagnosen empfiehlt sich für die Überwachung des gesunden Zustandes bis zur Fehlerfeststellung der Einsatz von Fehlergasfrühwarngeräten. Diese müssen sowohl gelöste als auch im Gassammelrelais frei enthaltene Fehlergase messen können.

Bisher bekannte technische Lösungen für Fehlergasfrühwarngeräte an flüssigkeitsgefüllten Hochspannungsanlagen können nur gelöste Fehlergase messen. Dazu wird Flüssigkeit an nur gasdurchlässige Membranen herangeführt, hinter denen Sensoren für brennbare Gase

(US 3866460) bzw. Wasserstoff (US 4058373) angeordnet sind. Werden vorgegebene Grenzwerte erreicht, gilt die Hochspannungsanlage als auffällig und muss analytisch aufwendiger überwacht werden.

Neben dem fehlenden Buchholzgas sind weitere Nachteile zu nennen: keine Online-Kalibrierbarkeit, Flüssigkeitslecks an der Membran, begrenzte Lebensdauer. Damit sind Fehlerfeststellungen weder lückenlos noch zuverlässig möglich.

Bekannt ist ferner ein Verfahren zur Kontrolle des Betriebsverhaltens von ölisolierten Hochspannungsgeräten, insbesondere Großtransformatoren, die mit einem mit der Außenluft in Verbindung stehenden Ausgleichsgefäß ausgerüstet sind, bei denen das Betriebsverhalten durch die Analyse des im Luftraum des Ausgleichsgefäßes vorhandenen Gasgemisches auf den Fehlergasgehalt, z.B. durch die Wasserstoffkonzentrationsüberwachung bestimmt wird (DD 148 688).

### Offenbarung der Erfindung

### Technische Aufgabe

Aufgabe der Erfindung ist es, an die mit Flüssigkeit gefüllten Hochspannungsgeräte eine als Fehlergasfrühwarngerät einsetzbare Messeinrichtung so zu installieren und zu betreiben, dass an derselben eine im Aktivteil des Transformators einsetzende Fehlergasemission lückenlos und zuverlässig festgestellt werden kann.

### Technische Lösung

Für die kostengünstige Realisierung der Erfindung ist von Bedeutung, dass das Fehlergas Wasserstoff für die gestellte Aufgabe zwei besonders vorteilhafte Eigenschaften besitzt, Fehleruniversalität und geringe Löslichkeit in der Flüssigkeit. Dieser Wasserstoff tritt im Fehlerfall bevorzugt aus der Flüssigkeit in den Gasraum des Ausdehners über, was in Kenntnis der Transport- und Austauschprozesse sowie mit einer leistungsfähigen Wasserstoffanalytik zur Fehlerfeststellung genutzt werden kann.

Komplettiert werden soll die Überwachung dadurch, dass auch das Buchholzgas in die Überwachung auf das Fehlergas Wasserstoff einbezogen wird. Gerade die Kombination beider Überwachungsaufgaben sichert eine lückenlose und zuverlässige Fehlerfeststellung. Besonders effektiv ist die Zusammenführung auf ein gemeinsames Messgerät sowie die Ableitung objektiver Grenzwerte.

Damit kann die Aufgabe der Erfindung erstmals ausschließlich über frei vorhandene Gase zuverlässig realisiert werden.

Die erfindungsgemäße Lösung ergibt sich aus den Ansprüchen 1 und 3. Vorteilhafte Ausführungen sind in den Unteransprüchen angegeben.

### Kurze Beschreibung der Zeichnungen

Die Erfindung soll nachfolgend anhand eines Ausführungsbeispieles erläutert werden. In den zugehörigen Zeichnungen zeigen

Fig. 1 das Grundprinzip der gasanalytischen Überwachung an einer flüssigkeitsgefüllten Hochspannungsanlage,

Fig. 2 die Installation einer Online-Messeinrichtung an einem Öltransformator mit Buchholz-Relais.

### Ausführungsform(en) der Erfindung

In Fig. 1, die bereits in der Beschreibungseinleitung erläutert wurde, ist mit 1 das Innere der flüssigkeitsgefüllten Hochspannungsanlage, mit 2 das Gassammelrelais und mit 3 der Ausdehner bezeichnet. Aus dem Gasraum 4 des Ausdehners 3 führt eine Atmungsleitung 5 in die Atmosphäre.

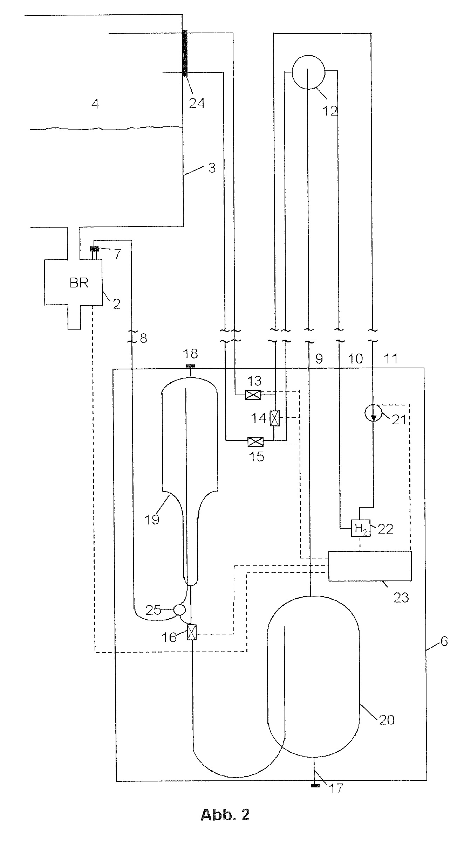

In Fig. 2 befindet sich die Online-Messeinrichtung 6 vorteilhafterweise in Bedienhöhe am Transformator oder seiner unmittelbaren Nähe und ist über metallische Rohrleitungen mit dem Buchholz-Relais 2 und dem Ausdehner 3 verbunden. Die Leitung 8 führt zum Entlüftungshahn 7 des Buchholz-Relais 2. Leitung 9 mündet in einem Gasmischgefäß 12, welches sich immer oberhalb des Ölspiegels im Ausdehner 3 befinden muss. Leitung 10 führt ebenfalls zum Gasmischgefäß 12 und von dort weiter über ein Schaltventil 15 in den Gasraum 4 des Ausdehners 3. Leitung 11 verläuft direkt zum Gasraum 4 über Schaltventil 13 und ragt oberhalb des Ölspiegels deutlich in den Gasraum 4 hinein. Die versetzten Enden der Leitungen 10 und 11 garantieren so beim Betrieb der Pumpe 21 eine gute Durchmischung im Gasraum 4. Die Leitungen 10 und 11, die vorteilhaft durch einen Flansch 24 geführt werden können, sind unmittelbar vor den Schaltventilen 13 und 15 mit Schaltventil 14 überbrückbar. Die Zuleitung 11 führt über die Gaspumpe 21 zum Wasserstoffsensor 22. Dieser, die Gaspumpe 21 sowie alle Schaltventile 13 - 16 sind mit der Steuer- und Auswerteeinheit 23 verbunden. Am Wasserstoffsensor 22 ist die Rückleitung 10 angeschlossen. Die Leitungen 8 und 9 in der Online-Messeinrichtung 6 sind über Separiergefäß 19 und Auffanggefäß 20 miteinander verbunden.

Gefäß 19 ist direkt oberhalb von Gefäß 20 angebracht.

Beide Gefäße sind über Schaltventil 16 miteinander so verbunden, dass der Ablauf vom Separiergefäß 19 zum Zulauf des Auffanggefäßes 20 führt. Das zylindrische Separiergefäß 19 besitzt den Probenahme- sowie Entlüftungshahn 18 und ist im unteren Teil verjüngt. Das Auffanggefäß 20 besitzt einen Entleerungshahn 17. Bevor Leitung 8 in das Separiergefäß 19 eintritt, passiert sie den Flüssigkeit/Gas-Trenner 25, der eine seitliche Verbindung zum Schaltventil 16 hat.

Nach der kompletten Installation der Messeinrichtung 6 mit allen Rohrleitungen wird der Entlüftungshahn 7 des Buchholz-Relais 2 geöffnet. Im Ausgangszustand sind an den Medienleitungen und an der Messeinrichtung alle Hähne und Schaltventile geöffnet. Damit fließt das Öl über Leitung 8 in den Flüssigkeit/Gas-Trenner 25 und von dort in das Auffanggefäß 20. Mit dem beginnenden Ölaustritt aus Hahn 17 wird Schaltventil 16 manuell geschlossen. Nun beginnt sich das Separiergefäß 19 zu füllen. Mit dem Ölablauf über Hahn 18 wird dieser geschlossen. Tritt aus Hahn 17 kein Öl mehr aus, wird dieser geschlossen. Zuletzt wird Schaltventil 14 manuell geschlossen.

### Beginn der Normalüberwachung

Die Messeinrichtung 6 wird über die Steuer- und Auswerteeinheit 23 in Betrieb gesetzt, es beginnt die Überwachung des aus dem Gasraum 4 abgeleiteten Messgases auf das Fehlergas Wasserstoff.

Über das Rohrleitungsende 11, das horizontal ca. 1 m weiter in den Gasraum 4 hineinragt als das Ende der Rohrleitung 10, wird dem Wasserstoffsensor 22 Messgas mit einem Durchsatz von z. B. 20 Liter/Stunde mittels der Pumpe 21 zugeführt.

Der Messbereich des Wasserstoffsensor 22 liegt im Bereich 10 - 5000 ppm. Zur Sicherung der Langzeitstabilität werden periodisch und automatisch Kalibrierungen durchgeführt. Die Messeinrichtung 6 kann sowohl kontinuierlich als auch periodisch betrieben werden. Die Unterscheidung des gesunden vom auffälligen Zustand des Transformators wird von statistisch gesicherten Grenzwerten für im Öl gelösten Wasserstoff abgeleitet. Zur Übertragung auf die Verhältnisse im Gasraum 4 wird ein Modell des Gashaushaltes von luftatmenden Öltransformatoren benutzt.

### Umschaltung auf Überwachung des Buchholzgases auf das Fehlergas Wasserstoff

Tritt ein Gasalarm im Buchholzrelais durch aufsteigende Fehlergasblasen auf, geht diese Information über die Signalleitung vom Buchholz-Relais 2 direkt an die Steuer- und Auswerteeinheit 23. Von dieser erfolgt die Abschaltung der Gaspumpe 21, das Öffnen von Schaltventil 16 und das Schließen von Schaltventil 13. Jetzt beginnt der Ölzulauf in das Auffanggefäß 20. Die dabei verdrängte Luft gelangt über die Leitung 9 in das Mischgefäß 12 und von dort in den Gasraum 4. Gelangt über Leitung 8 Buchholzgas in den Flüssigkeit/Gas-Trenner 25, wird es über die Steigleitung 8 in das Separiergefäß 19 transportiert. Hat das Buchholzgas vollständig das Öl im Separiergefäß 19 verdrängt, erfolgt der Eintritt des Buchholzgases in das Auffanggefäß 20. Zu diesem Zeitpunkt sollte das Auffanggefäß 20 zu ca. 90 % ölgefüllt sein. Das Buchholzgas gelangt dann über Leitung 9 auch in das Mischgefäß 12. Wenn das gesamte Buchholzgas aus dem Buchholz-Relais 2 abtransportiert ist, geht der obere Schwimmer des Buchholz-Relais in seine obere Endlage zurück und das Signal wird zur Steuer- und Auswerteeinheit 23 übertragen. Es beginnt der Ölspiegel im Auffanggefäß 20 in die Leitung 9 hinein zu steigen, bis er die Höhe des Ölspiegels im Ausdehner 3 unterhalb des Gasmischgefäßes 12 erreicht hat. Die Steuer- und Auswerteeinheit 23 wartet dies seit der Signalrücknahme über eine Zeitvorgabe ab und schaltet dann die Schaltventile 15 und 16 zu sowie das Schaltventil 14 auf. Die Gaspumpe 21 schaltet wieder ein und ein interner Gaskreislauf in den Leitungen 10, 11 zwischen Gasmischgefäß 12 und Wasserstoffsensor 22, in dem der Buchholzgasanteil aus dem Mischgefäß 12 ca. 10 % beträgt, beginnt mit der Homogenisierung des Gasgemisches. Nach einer erneuten Zeitvorgabe steht ein Messsignal aus dem Wasserstoffsensor 22 an, welches mit einem Grenzwert für Wasserstoff verglichen wird. Die Aussage lautet dann: Fehlergas oder Luft.

### Entnahme einer Probe des Buchholzgases

Zur Entnahme einer Probe des Buchholzgases aus dem Separiergefäß 19 erfolgt mit dem Schließen des Schaltventils 16 im Separiergefäß 19 ein Druckanstieg um die Höhe der statischen Ölsäule bis zum Ölspiegel des Ausdehners 3. Synchron dazu steigt der Ölspiegel im verjüngten Teil des Separiergefäßes 19 dadurch an, dass Öl über den Flüssigkeit/Gas-Trenner 25 aus Richtung Schaltventil 16 zugeführt wurde. Um für die Standzeit des Buchholzgases im Separiergefäß 19 den Gasaustausch mit dem Öl zu minimieren, hat das Separiergefäß 19 die im unteren Teil verjüngte Form (ca. ¹/₃ des Gefäßvolumens).

Während der Gasentnahme über Hahn 18 läuft das Öl ebenfalls über den Flüssigkeit/Gas-Trenner 25 von unten in das Separiergefäß 19 nach.

Ist die Probenahme über Hahn 18 beendet, wird dieser geschlossen, nachdem Öl abläuft.

Vorher oder nachher ist über Hahn 17 das Auffanggefäß 20 vollständig zu entleeren und Hahn 17 zu schließen.

### Rückschaltung auf Normalüberwachung

Über die Steuer- und Auswerteeinheit 23 wird die Messeinrichtung 6 wiederum in Betrieb gesetzt. Die Schaltventile 13 und 15 gehen auf und Schaltventil 14 zu, es beginnt wieder die Überwachung des Gasraumes 4 des Ausdehnungsgefäßes 3 auf das Fehlergas Wasserstoff.

## Patentansprüche

1. Verfahren zur Fehlergasüberwachung von flüssigkeitsgefüllten Hochspannungsanlagen, deren Flüssigkeit über ein Gassammelrelais und Ausdehnungsgefäß mit der atmosphärischen Luft in Verbindung steht, **gekennzeichnet durch** die Messung und Auswertung des Fehlergases Wasserstoff
a) in dem aus dem Gasraum (4) des Ausdehnungsgefäßes (3) abgeleiteten Messgas und
b) in dem bei einem Gasalarm des Gassammelrelais (2) aus diesem abgeleiteten Buchholzgas
in einer einheitlichen Online-Messeinrichtung (6) mit einem Wasserstoffsensor (22), wobei bei einem Gasalarm des Gassammelrelais (2) die Überwachung des Gasraumes (4) des Ausdehnungsgefäßes (3) unterbrochen, auf eine Messung und Auswertung auf das Fehlergas Wasserstoff in dem abgeleiteten Buchholzgas umgeschaltet und nach Vorliegen eines Prüfergebnisses auf die Überwachung des Gasraumes (4) zurückgeschaltet wird und dass bei einem Prüfergebnis "Fehlergas Wasserstoff" im Buchholzgas zur Vollanalyse eine Probenahme durchgeführt wird.

2. Verfahren nach Anspruch 1, **dadurch gekennzeichnet, dass** das bei einem Gasalarm auftretende Buchholzgas in ein mit einer Probenahmeeinrichtung (18) versehenes Separiergefäß (19) strömt und von diesem nach Abschaltung der Überwachung des Ausdehnungsgefäßes (3) zur Messung und Auswertung auf das Fehlergas Wasserstoff über ein Gasmischgefäß (12) zum Wasserstoffsensor (22).

3. Vorrichtung zur Fehlergasüberwachung von flüssigkeitsgefüllten Hochspannungsanlagen, deren Flüssigkeit über ein Gassammelrelais und Ausdehnungsgefäß mit der atmosphärischen Luft in Verbindung steht, **gekennzeichnet durch** eine in Bedienhöhe der Hochspannungsanlage angeordnete Online-Messeinrichtung (6) mit einem Wasserstoffsensor (22) zur Messung und Auswertung des Fehlergases Wasserstoff in dem aus dem Gasraum (4) des Ausdehnungsgefäßes (3) abgeleiteten Messgas und in dem bei einem Gasalarm auftretenden Buchholzgas, mit einer Probenahmeeinrichtung (18) zur Vollanalyse des Buchholzgases sowie einer Steuer- und Auswerteeinheit (23) mit folgenden Funktionen:
3.1. Messung und Auswertung des Fehlergases Wasserstoff in dem aus dem Gasraum (4) abgeleiteten Messgas;
3.2. Unterbrechung dieser Funktion beim Auftreten eines Gasalarms im Gassammelrelais, Steuerung der Ableitung des Buchholzgases und Messung sowie Auswertung auf das Fehlergas Wasserstoff;
3.3. Umschaltung auf die Funktion 3.1.

4. Vorrichtung nach Anspruch 3, **dadurch gekennzeichnet, dass** zum Transport des Messgases zum Wasserstoffsensor (22) eine Gaspumpe (21) vorgesehen ist, die über eine Leitung (11) entweder mit dem Gasraum (4) des Ausdehnungsgefäßes (3) oder nach Umschaltung auf Messung und Auswertung des Buchholzgases mit einem Gasmischgefäß (12) in Verbindung steht.

5. Vorrichtung nach Anspruch 3 und 4, **dadurch gekennzeichnet, dass** die Online-Messeinrichtung (6) ein mit der Probenahmeeinrichtung (18) versehenes Separiergefäß (19) zur Sammlung und Aufbewahrung des bei einem Gasalarm aus dem Gassammelrelais (2) abgeströmten Buchholzgases aufweist und das Separiergefäß (19) nach Abschaltung der Überwachung des Gasraumes (4) des Ausdehnungsgefäßes (3) mit dem Gasmischgefäß (12) in Verbindung steht.

6. Vorrichtung nach Anspruch 3 bis 5, **dadurch gekennzeichnet, dass** die Online-Messeinrichtung (6) unterhalb des Separiergefäßes (19) ein mit diesem über ein Schaltventil (16) verbundenes Auffanggefäß (20) aufweist, wobei der Ablauf vom Separiergefäß (19) zum Zulauf des Auffanggefäßes (20) führt und dass vom Auffanggefäß (20) eine vertikale Ausgleichsrohrleitung (9) zu dem Gasmischgefäß (12) führt.

7. Vorrichtung nach den Ansprüchen 3 bis 6, **dadurch gekennzeichnet, dass** die Steuer- und Auswerteeinheit (23) mit dem Wasserstoffsensor (22), dem Gassammelrelais (2), den Schaltventilen (13-16) in den Leitungen sowie einer in der Zuleitung zum Wasserstoffsensor (22) angeordneten Gaspumpe (21) in Verbindung steht.

8. Vorrichtung nach den Ansprüchen 3 bis 7, **dadurch gekennzeichnet, dass** in der Leitung (8) vom Gassammelrelais (2) zur Online-Messeinrichtung (6) vor dem Eintritt in das Separiergefäß (19) ein Flüssigkeit-/Gas-Trenner (25) angeordnet ist, der eine seitliche Verbindung zum Schaltventil (16) besitzt.

9. Vorrichtung nach den Ansprüchen 3 bis 8, **dadurch gekennzeichnet, dass** das Separiergefäß (19) zur Minimierung des Gasaustausches mit dem Öl für die Standzeit des Buchholzgases im Separiergefäß im unteren Teil eine verjüngte Form hat.

10. Vorrichtung nach Anspruch 3, **dadurch gekennzeichnet, dass** die Leitung (11) für die Ableitung des Messgases aus dem Ausdehner (3) oberhalb des Ölspiegels weiter in den Gasraum (4) hineinragt als die unterhalb derselben angeordnete Rückführleitung (10).
